# EUROPÄISCHE PATENTANMELDUNG

(11) **EP 4 686 941 A1**
(43) Veröffentlichungstag der Anmeldung: **04.02.2026**
(21) Anmeldenummer: 25175266.3
(22) Anmeldetag: 09.05.2025
(51) Int. Cl.: G01N 33/15

(54) **TESTGERÄT ZUM VERMESSEN VON KLEINVOLUMIGEM SCHÜTTGUT**

(30) Priorität: 01.08.2024 DE 102024121985
(71) Anmelder: Kraemer, Thilo, 64291 Darmstadt (DE)
(72) Erfinder: Kraemer, Thilo, 64291 Darmstadt (DE)
(74) Vertreter: Hamel, Armin

(57) **Zusammenfassung**

Beschrieben wird ein Testgerät (1) zum Vermessen von kleinvolumigem Schüttgut. Dieses Testgerät (1) weist in einem Arbeitsraum (36) eine Messvorrichtung (23) auf, die mindestens eine Messstation (34) umfasst, in der das Schüttgut vermessen werden kann. In dem Arbeitsraum (36) des Testgeräts (1) ist zusätzlich ein Umweltinformationssystem untergebracht, das mindestens einen Sensor aufweist, mit dem eine bestimmte Umweltgröße gemessen werden kann und diese gemessene Umweltgröße in ein elektrisches Signal umwandelt. Das Computersystem prüft anhand der aus dem mindestens einem elektrischen Signal erhaltenen Daten, ob die zumindest eine ermittelte Umweltgröße innerhalb eines vorgegeben Wertebereichs liegt oder nicht. Zudem kann das Computersystem anhand den aus der zumindest einen gemessenen Umweltgröße erhaltenen Daten die Messwerte für eine bestimmte Schüttgutsorte der mindestens einen gemessenen Umweltgröße zuordnen. Dadurch werden Messungen einer bestimmten Schüttgutsorte in einem bestimmten Testgerätetyp vergleichbar und reproduzierbar.

## Beschreibung

Die Erfindung betrifft ein Testgerät zum Vermessen von kleinvolumigem Schüttgut nach dem Oberbegriff des Patentanspruchs 1.

Mit einem Testgerät zum Vermessen von kleinvolumigem Schüttgut kann kleinvolumiges Schüttgut, wie zum Beispiel Körner, Oblongs oder Tabletten, hinsichtlich Qualität, Zusammensetzung und Eigenschaften, wie beispielsweise der Härte oder der Masse, getestet werden. Dazu weist das Testgerät eine Messvorrichtung mit mindestens einer Messstation auf.

Ein solches Testgerät zum Vermessen von kleinvolumigem Schüttgut ist beispielsweise aus DE 10 2022 100 828 A1 bekannt. Das Testgerät umfasst eine Anordnung zur Vereinzelung, sowie eine unterhalb der Anordnung zur Vereinzelung angeordnete Messvorrichtung, wobei die Messvorrichtung mehrere Messstationen umfasst, mit der ein Schüttgut vermessen werden kann. Zwischen der Anordnung zur Vereinzelung und der Messvorrichtung ist eine Transportvorrichtung vorgesehen, mit der das Schüttgut den Messstationen der Messvorrichtung zugeführt werden kann.

Das Vermessen des Schüttguts in der Messvorrichtung des in DE 10 2022 100 828 A1 beschriebenen Testgeräts erfolgt jedoch, ohne dass äußere Messgrößen (im Folgenden auch Umweltgrößen, wie zum Beispiel Luftfeuchtigkeit oder Temperatur) berücksichtigt werden. Diese Umweltgrößen können jedoch die Messungen beeinflussen und so zu falschen bzw. nicht reproduzierbaren Messergebnissen führen.

Aus CN 209372832 U ist ein Testgerät bekannt, das aus einem Kopf, einem Kasten, einem Wassertank und einer Basis besteht. Universalräder sind gleichmäßig an den vier Ecken des unteren Endes der Basis angeordnet. Dabei ist am oberen Ende der Basis ein Kastenkörper angeordnet. Ein Maschinenkopf ist über eine Stützstange am oberen Ende des Kastenkörpers angeordnet, wobei ein Ein-Chip-Mikrocomputer auf einer Seite des Maschinenkopfinneren installiert ist. Heizblöcke sind gleichmäßig auf einer Seite des Wassertankinneren angeordnet und eine Halbleiter-Kühlplatte ist auf der anderen Seite des Wassertankinneren angeordnet. Ein Temperatursensor ist so angeordnet, dass er bei zu hoher oder zu niedriger Temperatur ein Temperatursignal erkennt und an den Ein-Chip-Mikrocomputer sendet. Dieser verarbeitet das Signal und sendet es dann zum Heizblock oder zur Halbleiter-Kühlplatte zum Heizen oder Kühlen. Dadurch wird eine intelligente Temperaturregelung des im Wassertank befindlichen Wassers ermöglicht.

Der Temperatursensor dient jedoch nur dazu zu prüfen, ob das Wasser die richtige Temperatur hat. Hat das Wasser nicht die gewünschte Temperatur, so wird das Wasser entweder abgekühlt oder aufgeheizt. Abgesehen davon, dass dieses Gerät nur einen Sensor besitzt, dient der Sensor nur dazu, direkt auf die Umgebung, nämlich die Temperatur des Wassers, Einfluss zu nehmen

Es ist mit dem in CN 209372832 U beschrieben System allerdings nicht möglich, für eine bestimmte Schüttgutsorte und einen bestimmten Testgerätetyp vergleichbare und reproduzierbare Messergebnisse zu erhalten.

Es wird daher ein Testgerät zum Vermessen von Schüttgut beschrieben, das eine Messvorrichtung aufweist, die mindestens eine Messstation umfasst. Diese Messvorrichtung ist in einem mit einem gasförmigen Fluid, wie beispielsweise Luft, gefüllten Arbeitsraum angeordnet. In der mindestens einen Messstation wird das Schüttgut einer bestimmten Sorte vermessen, zum Beispiel ein Oblong oder eine Tablette. Das Testgerät weist in dem Arbeitsraum ein Umweltinformationssystem auf, das mindestens einen Sensor und/oder mindestens einen Multisensor aufweist, mit dem eine bestimmte Umweltgröße gemessen werden kann. Diese gemessene Umweltgröße kann in ein elektrisches Signal umgewandelt und dieses elektrische Signal an ein Computersystem, bestehend aus Hardware und Software, weitergeleitet werden. In dem Computersystem findet die Auswertung mittels der Software statt.

Vorzugsweise weist die Messvorrichtung des Testgeräts mehrere Messstationen auf. Dadurch können für jedes Schüttgut einer bestimmten Sorte verschiedene Messwerte für bestimmte Eigenschaften bzw. physikalische Größen des Schüttguts, wie zum Beispiel die Länge des Schüttguts, die Breite des Schüttguts, die Masse, die Härte und/oder die Zusammensetzung des Schüttguts, erhalten werden.

Das Testgerät umfasst ein Umweltinformationssystem mit mindestens einem Sensor oder mindestens einem Multisensor, vorzugsweise jedoch mit mehreren Sensoren oder Multisensoren. Dieser mindestens eine Sensor misst eine bestimmte Umweltgröße in dem Arbeitsraum des Testgeräts, zum Beispiel die Temperatur, die Luftfeuchtigkeit oder die Staubdichte. Weist das Umweltinformationssystem mehrere Sensoren, einen Multisensor oder mehrere Multisensoren auf, können verschiedene Umweltgrößen gemessen werden. Auch kann das Umweltinformationssystem mehrere Sensoren bzw. Multisensoren aufweisen, die in dem Testgerät an verschiedenen Orten angeordnet sind und ein und dieselbe Umweltgröße messen, zum Beispiel die Temperatur, um zu dieser Umweltgröße einen Mittelwert bilden zu können. Die gemessenen Umweltgrößen werden in dem Umweltinformationssystem in elektrische Signale umgewandelt und an ein Computersystem weitergeleitet. Jeder Sensor misst also eine bestimmte Umweltgröße, wobei diese Umweltgröße in ein elektrisches Signal umgewandelt und an das Computersystem weitergeleitet wird.

Das Computersystem des Testgeräts wertet nun die erhaltenen Daten aus.

Dazu prüft das Computersystem anhand den aus dem mindestens einem elektrischen Signal, das aus einer Umweltgröße erhaltenen wurde, generierten Daten, ob die zumindest eine ermittelte Umweltgröße innerhalb eines vorgegeben Wertebereichs liegt oder nicht. Diese ermittelten Umweltgrößen bilden die Rahmenbedingungen, unter denen das Schüttgut vermessen wird. Weiterhin kann das Computersystem die mindestens eine Umweltgröße den erhaltenen Messwerten für einen bestimmten Schüttgutsorte zuordnen.

Das Testgerät wird im Folgenden anhand von Figuren näher erläutert. Es zeigen:
Figur 1 eine Seitenansicht eines Testgeräts für das Vermessen von kleinvolumigem Schüttgut und
Figur 2 eine Draufsicht auf das Testgerät nach Schnitt durch die Ebene A-A.

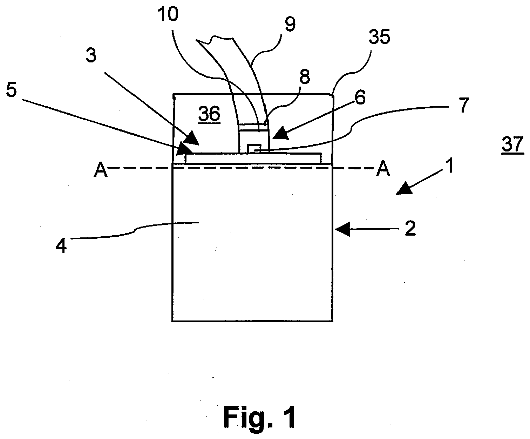

Figur 1 zeigt eine Seitenansicht eines Testgeräts 1 zum Vermessen von Schüttgut. Bei dem kleinvolumigen Schüttgut handelt es sich beispielsweise um ein medizinisches Produkt, das zum Beispiel in Form einer Tablette, eines Oblongs oder eines Korns vorliegen kann.

Das Schüttgut besteht aus der gleichen Sorte (zum Beispiel Oblongs), womit es sich um einen bestimmten Schüttgutsorte handelt. Es wird mit dem Testgerät also in einer ersten Messreihe ein Schüttgut einer bestimmten Sorte vermessen. Nach dieser Messreihe kann in weiteren, darauffolgenden Messreihen Schüttgut einer anderen Sorte oder aber der gleichen Sorte vermessen werden.

Das Testgeräts 1 umfasst einen unteren Abschnitt 2 auf dem eine Anordnung zur Vereinzelung 3 angeordnet ist. Der untere Abschnitt 2 ist von einem Gehäuse 4

umgeben, weshalb das Innenleben des unteren Abschnitts 2 in der Figur 1 nicht zu sehen ist.

Die Anordnung zur Vereinzelung 3 umfasst ein als Vibrationsplatte ausgebildetes Plattenelement 5 sowie einen einfach abzunehmenden Vorratsbehälter 6, in dem Schüttgut bevorratet werden kann. Die Vibrationsplatte wird über einen in der Figur 1 nicht zu sehenden Vibrationsantrieb in Bewegung versetzt. Dieser Vibrationsantrieb ist in dem unteren Abschnitt 2 angeordnet. Der Vorratsbehälter 6 kann mit der Vibrationsplatte 5 entweder fest verbunden sein, oder von dieser Vibrationsplatte 5 entkoppelt sein.

Der Vorratsbehälter 6 umfasst eine kleine Öffnung 7, über die das Schüttgut der gleichen Sorte auf die Vibrationsplatte 5 gelangen kann. Schüttgut ist in der Figur 1 nicht dargestellt. Über eine obere Zuführungsöffnung (in dieser Ansicht nicht zu sehen), die sich in einem oberen Bereich 10 des Vorratsbehälters 6 befindet, kann Schüttgut über eine Schüttgutzuführung 9 in den Vorratsbehälter 6 eingebracht werden. Die Schüttgutzuführung 9 ist durch eine Abdeckung 35 geführt, die dazu eine nicht zu sehende Öffnung besitzt, durch die die Schüttgutzuführung 9 geführt ist. Die Abdeckung 35 sitzt auf dem unteren Abschnitt 2, wobei der Raum innerhalb der Abdeckung 35 einen Arbeitsraum 36 bildet, in dem das Schüttgut vermessen wird. Dieser Arbeitsraum 36 kann gegenüber einer Außenumgebung 37 durch nicht dargestellte Dichtungen abgedichtet sein. Dieser Arbeitsraum ist mit einem gasförmigen Fluid gefüllt, wie zum Beispiel Luft oder Inertgas. Möglich wäre auch, dass der Arbeitsraum mit einem Inertgas gefüllt ist.

In Figur 1 besitzt das Testgerät 1 eine Abdeckung 35, wobei der Raum innerhalb der Abdeckung 35 den Arbeitsraum 36 bildet. Es versteht sich, dass bei Weglassen der Abdeckung 35 ebenfalls ein Arbeitsraum gebildet wird, nämlich durch das Umfeld. Es würde sich somit um einen gegenüber dem Arbeitsraum 36 erweiterten Arbeitsraum handeln, der aus dem Arbeitsraum 36 und der Außenumgebung 37 besteht. In diesem erweiterten Arbeitsraum 36, 37 kann sich Luft befinden.

Damit Schüttgutzuführungen verschiedenen Typs schnell und einfach am Vorratsbehälter 6 angebracht werden können, besitzt der Vorratsbehälter 6 im oberen Bereich 10 einen Normanschluss 8. Dieser Normanschluss 8 kann beispielsweise eine Schelle sein, mit der die entsprechende Schüttgutzuführung, wie hier die Schüttgutzuführung 9, an dem Vorratsbehälter 6 befestigt werden kann.

Ein Umbau und damit eine Anpassung der Anordnung zur Vereinzelung an unterschiedliche Schüttgutzuführungen ist daher nicht erforderlich. Dadurch kann das Testgerät 1 an verschiedenen Standorten eingesetzt werden, also auch an Standorten, an denen Platz für einen Umbau bzw. Anpassung der Anordnung zur Vereinzelung nicht vorhanden ist oder aber Werkzeug zum Umbau bzw. Anpassung nicht verfügbar ist.

Bei der in Figur 1 dargestellten Schüttgutzuführung 9 handelt es sich um einen Schlauch, über den Schüttgut in den Vorratsbehälter 6 eingebracht werden kann. Da der Vorratsbehälter 6 einen Normanschluss 8 besitzt, kann auch eine andere Schüttgutzuführung, zum Beispiel ein Trichter, einfach und schnell an dem Vorratsbehälter 6 angebracht werden.

Da der Vorratsbehälter 6 einfach abzunehmen ist, kann der Vorratsbehälter 6 auch separat befüllt werden und anschließend wieder auf das Testgerät 1 gesetzt werden.

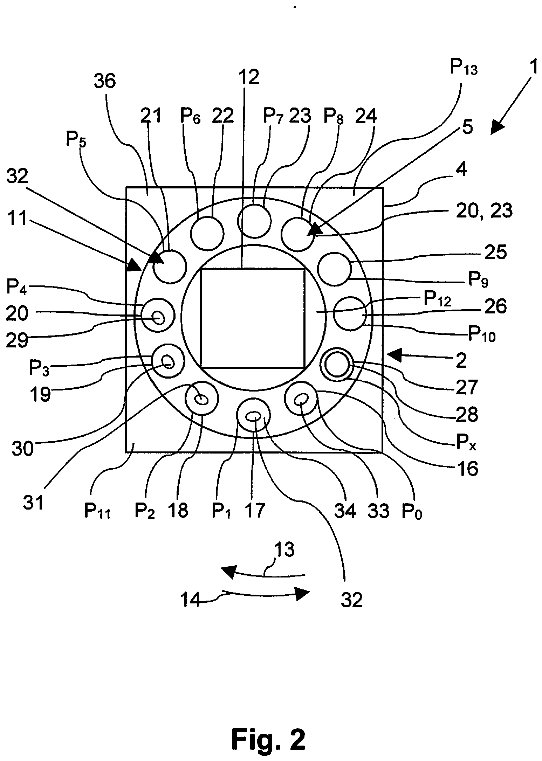

Figur 2 zeigt eine Draufsicht auf den unteren Abschnitt 2 des Testgeräts 1 nach einem Horizontalschnitt entlang einer Ebene A-A (siehe Figur 1) ohne die Abdeckung 35. Eine Transportvorrichtung 11 umgibt den Vibrationsantrieb 12 von allen Seiten und kann um diesen Vibrationsantrieb 12 in Richtung des Pfeils 13 vorbewegt bzw. in Richtung des Pfeils 14 zurückbewegt werden. Die Transportvorrichtung 11 bewegt sich dabei um den Vibrationsantrieb 12.

Mit dem Vibrationsantrieb 12 kann die in Figur 1 zu sehende Vibrationsplatte 5 in Vibration versetzt werden.

Die Transportvorrichtung 11 ist in Figur 2 als Transportstern ausgebildet und wird oberhalb eines Bodens 15 des unteren Abschnitts 2 bewegt.

Die Transportvorrichtung 11 weist mehrere Kammern 16 bis 27 auf, die das Schüttgut aufnehmen können. Jeweils ein als Oblong ausgebildetes Schüttgut 29 bis 33 befindet sich bereits in den Kammern 16 bis 20. Die anderen Kammern 21 bis 27 sind leer. Bei der Kammer 16 handelt es sich um eine erste Kammer 16, in der das Schüttgut eingebracht wird, nachdem es vereinzelt wurde. Folglich wurde das Schüttgut 33 gerade in die Kammer 16 eingebracht.

Das in der Kammer 16 angeordnete Schüttgut 33 befindet sich in einer Ausgangsposition P0. Mit einem nicht dargestellten Detektor wird festgestellt, ob in die Kammer 16 nur ein Schüttgut oder aber mehrere Schüttgüter eingebracht wurden. Dieser Detektor kann eine Videokamera sein. Wurde festgestellt, dass sich nur ein Schüttgut, nämlich das Schüttgut 33, in der Kammer 16 befindet, so wird das Schüttgut 33 mittels der Transportvorrichtung 11 weiter in Richtung des Pfeils 13 bis zu der Position Px bewegt. In der Position Px befindet sich im Boden 15 eine Öffnung 28, unter der ein Abfallbehälter (nicht zu sehen) angeordnet ist, so dass das in der Kammer 21 befindliche Schüttgut oder Bruchstücke eines Schüttguts in den Abfallbehälter fallen und so entsorgt werden können.

Wird das Schüttgut 33 von der der Position P0 in Richtung der Position Px transportiert, so gelangt das Schüttgut 33 von der Position P0 zuerst zu der Position P1. In der Position P1 befindet sich im Boden 15 eine erste Messstation 34 einer Messeinrichtung 23 in Form einer Waage 34. Mit dieser Waage 34 wird ermittelt, ob es sich tatsächlich nur um ein Schüttgut handelt, das sich in der Kammer 16 befindet. Würden sich in der Position P1 nur Bruchstücke oder gar mehrere Schüttgüter befinden, so würde die Transportvorrichtung 11 in Richtung des Pfeils 14 zurückfahren, bis die Kammer 16 die Position Px erreicht hat. Dort werden Bruchstücke oder die mehreren Schüttgüter entsorgt. Sodann wird die Vorrichtung 11 wieder vor in Richtung des Pfeils 13 gefahren, bis die Kammer 16 wieder die Ausgangsposition P0 erreicht hat. In dieser Ausgangsposition P0 wird die Kammer 16 erneut mit einem Schüttgut bestückt.

Ist festgestellt worden, dass sich nur ein Schüttgut in der Position P1 befindet, so wird das Schüttgut zu der Positionen Px transportiert, wobei dieses Schüttgut nacheinander die Positionen P2 bis P10 passiert.

Dabei kann im Boden 15 bei jeder der Positionen P2 bis P10 eine weitere Messstation der Messvorrichtung 23 angeordnet sein. Diese weiteren Messstationen sind der Übersicht halber nicht mit Bezugszeichen versehen. In der Position P10 ist vorzugsweise eine Härtemessvorrichtung angeordnet, in der nicht nur die Härte mittels eines Bruchtests, sondern auch die Breite und Länge des in der Härtemessvorrichtung (auch Bruchkammer genannt) angeordneten Schüttguts ermittelt werden kann. Dabei ist es möglich, in dem Boden 15 in den Positionen P2 bis P9 als Messstationen jeweils einen NIR-Sensor vorzusehen. Mit diesen NIR-Sensoren kann die Zusammensetzung des Schüttguts ermittelt werden. Jeder NIR-Sensor bildet damit eine Messstation und ist - wie auch die Waage 34 bei der Position P1 - Teil der Messvorrichtung 23. Beim Passieren dieser Positionen P2 bis P9 wird das Schüttgut 33 somit mittels NIR vermessen. Die Messvorrichtung 23 umfasst damit acht NIR-Sensoren. Denkbar ist auch, dass nur bei den Positionen P2 bis P5 im Boden 15 NIR-Sensoren als Messstationen vorgesehen sind und dass in den Positionen P6 bis P9 das Schüttgut mittels Videokameras vermessen wird. Dazu sind die Videokameras ebenfalls im Boden 15 angeordnet, wobei jede Videokamera eine Messstation bildet. In diesem Fall würde die Messvorrichtung 23 also ebenfalls zehn Messstationen aufweisen, nämlich eine Waage 34, vier Videokameras sowie vier NIR-Sensoren und eine Härtemessvorrichtung.

Die Härtemessvorrichtung kann sich allerdings auch der Position P9 befinden. In der darauffolgenden Position P10 kann eine Videokamera angeordnet sein, mittels der geprüft wird, ob mittels der Härtemessvorrichtung der Bruchtest korrekt durchgeführt wurde oder nicht.

Mit den NIR-Sensoren wird die chemische Zusammensetzung bzw. die Qualität des Schüttguts ermittelt. Mit den Videokameras wird insbesondere geprüft, ob tatsächlich nur ein Schüttgut in die Transportvorrichtung 11 gelangt ist, oder ob das Schüttgut die gewünschte Form hat.

Nachdem das Schüttgut die Messstationen P1 bis P10 passiert hat und vermessen wurde, wird es zu der Position Px transportiert, wo es über die Öffnung 28 in den Abfallbehälter fällt.

Der Übersicht halber sind die einzelnen Messstationen der Messvorrichtung 23, die sich in den Positionen P0 bis P10 befinden und in dem Boden 15 angeordnet sind, nicht mit Bezugszahlen versehen.

Um zu gewährleisten, dass mit der Messvorrichtung 23 bei der Vermessung des Schüttguts exakte und reproduzierbare Messergebnisse erhalten werden, umfasst das Testgerät 1 ein Umweltinformationssystem, das zumindest einen Sensor aufweist, mit dem eine bestimmte Umweltgrößen gemessen werden kann. Dieser mindestens eine Sensor des Umweltinformationssystems ist in dem Arbeitsraum 36 angeordnet. Dadurch können die Umweltgrößen in unmittelbarer Nähe des zu vermessenden Schüttguts gemessen werden (= Testumgebung). Vorzugsweise umfasst das Umweltinformationssystem mehrere Sensoren, wobei mit jedem Sensor des Umweltinformationssystems eine andere Umweltgröße gemessen werden kann. Bei diesen Umweltgrößen handelt es sich um physikalische oder chemische Eigenschaften. Insbesondere handelt es sich um physikalische Eigenschaften wie zum Beispiel Temperatur, Luftfeuchtigkeit, Luftdruck, Schallfeldgrößen oder Helligkeit. Daneben kann das Umweltinformationssystem auch Sensoren aufweisen, mit denen eine stoffliche Beschaffenheit der Umgebung qualitativ oder quantitativ erfasst werden kann. Zum Beispiel kann das Umweltinformationssystem hierzu einen Sensor aufweisen, mit dem die Dichte beziehungsweise die Menge an Staubpartikeln in der Luft erfasst werden kann.

Im Folgenden werden einige Sensoren und deren Einsatzgebiet näher beschrieben. Der Übersicht halber sind die Sensoren des Umweltinformationssystems in der Figur 2 nicht mit Bezugszeichen versehen.
- Sensor zum Messen der Temperatur (Temperatursensor):
   Mit diesem Sensor wird die Temperatur gemessen, die in dem Arbeitsraum 36 des Testgeräts 1 herrscht. Dieser Sensor kann in dem Gehäuse 4, beispielsweise in der Position P0, angeordnet sein, also in der Position, in der sich auch der Detektor befindet. Dieser Temperatursensor kann beispielsweise neben dem Detektor oder
   oberhalb des Bodens 15 angeordnet sein.

In dem Testgerät 1 können aber auch weitere Temperatursensoren angeordnet sein, beispielsweise in den Positionen P5 und P8. Die Temperatursensoren in diesen drei Positionen können dabei in dem Boden 15 oder aber auch oberhalb des Bodens 15 angebracht sein.

Die Temperatursensoren können aber auch irgendwo anders in dem Arbeitsraum 36 des Testgeräts 1 angeordnet sein, zum Beispiel an dem Boden 15 bei den Positionen P11 bis P13. Die Sensoren können aber auch anderswo im Arbeitsraum 36 liegen, da es lediglich wichtig ist, dass die Sensoren im Arbeitsraum 36 angeordnet sind. Sinnvoll ist es, diese Temperatursensoren in unmittelbarer Nähe der Messvorrichtung 23 anzuordnen, so dass besser nachvollziehbar ist, welchen Einfluss diese Umweltgröße auf die durch die Vermessung des Schüttguts erhalten Messergebnisse nimmt, als wenn die Temperatursensoren sehr weit weg von der Messvorrichtung 23 angeordnet wären.
- Sensor zum Messen der Luftfeuchtigkeit:
   Mit diesem Sensor wird die Luftfeuchtigkeit in dem Arbeitsraum 36 des Testgeräts 1 gemessen. Dabei können in dem Testgerät 1 auch mehrere Sensoren zum Messen der Luftfeuchtigkeit vorgesehen sein. Wie auch die Temperatursensoren können die Sensoren zum Messen der Luftfeuchtigkeit ebenfalls an verschiedenen Positionen in dem Arbeitsraum 36 des Testgeräts 1 angeordnet sein, wobei es ebenfalls sinnvoll ist, diese Sensoren in der Nähe der Messvorrichtung 23 anzuordnen. So kann ein solcher Sensor beispielsweise in der Position P12 angeordnet sein, sollte sich dort kein Temperatursensor befinden.

Das Anordnen dieser Sensoren in dem Testgerät 1 ist deshalb sinnvoll, weil die Luftfeuchtigkeit die durch die Vermessung des Schüttgut erhalten Messergebnisse beeinflussen und somit verfälschen kann. So kann das Schüttgut bei zu hoher Luftfeuchtigkeit beispielsweise Wasser aufnehmen, wodurch die Messergebnisse der Härtemessung, der Masse oder der qualitativen Vermessung des Schüttguts mittels NIR, insbesondere hinsichtlich der Zusammensetzung des Schüttguts, beeinflusst werden können.
- Sensor zum Messen des Luftdrucks:
   Druckluftschwankungen innerhalb des Arbeitsraums 36 können dazu führen, dass die Waage 34 zu Schwingungen angeregt wird, so dass diese das Schüttgut, dessen Masse ermittelt werden soll, nicht korrekt vermisst und so Messwerte erhalten werden, die verfälscht sind.
- Sensor zum Messen von vorhandenen Gasen
   Mit diesem Sensor können die verschiedenen Gase erfasst werden, die sich im Arbeitsraum befinden, wie zum Beispiel CO2, NOx oder SOx.
- Sensor zum Messen von Schallfeldgrößen:
   Auch Schallwellen können Einfluss auf das Wiegen des Schüttguts haben, weil Schallwellen die Waage 34 zu unerwünschten Schwingungen anregen können.
- Sensor zum Messen der Helligkeit:
   Ist es in dem Testgerät zu dunkel oder zu hell, so kann dies dazu führen, dass bei der Vermessung des Schüttguts mittels Videokameras sehr schlechte Messergebnisse, nämlich Bilder von schlechter Qualität, erhalten werden. Ist es beispielsweise in dem Testgerät zu dunkel oder zu hell, kann dies dazu führen, dass eine Videokamera die Form des Schüttguts nicht eindeutig erkennt. Schlimmstenfalls kann dies sogar dazu führen, dass nicht erkannt wird, ob sich in einer bestimmten Position, bei der Schüttgut mittels dieser Videokamera vermessen wird, tatsächlich Schüttgut vorhanden ist oder nicht. Zudem kann ein zu großer Lichteinfall bei den NIR-Sensoren die Messergebnisse verfälschen.
- Sensor zum Messen der Dichte an Staubpartikeln:
   Sind in dem Testgerät 1 zu viele Staubartikel vorhanden, so kann dies dazu führen, dass bei der Vermessung des Schüttguts mittels NIR-Sensoren oder Videokameras sehr schlechte Messergebnisse erhalten werden. Ist zum Beispiel zu viel Staub in dem Testgerät 1 vorhanden, kann dies dazu führen, dass eine Videokamera die Form des Schüttguts nicht genau erkennt.

Mit diesem Sensor können so auch die Masse und die Anzahl an Staubpartikeln ermittelt werden.

Anstelle der verschiedenen Sensoren kann auch ein Multisensor oder mehrere Multisensoren eingesetzt werden. Ein solcher Multisensor besteht aus mehreren unterschiedlichen Sensoren und kann zum Beispiel 3 bis 5 Sensoren umfassen. Das Umweltinformationssystem mit dem zumindest einen Sensor, mit dem eine bestimmte Umweltgröße gemessen werden kann, erfüllt zwei Funktionen, wobei das Umweltinformationssystem vorzugsweise mehrere Sensoren bzw. Multisensoren umfasst, mit denen verschiedene Umweltgrößen gemessen werden können:
1. Es kann bereits vor Beginn des Vermessens von Schüttgut einer bestimmten Sorte geprüft werden, ob die ermittelte Umweltgrößen (zum Beispiel die gemessene Temperatur, die Staubdichte oder die Helligkeit) innerhalb eines vorgegebenen Wertebereichs liegt. Liegen diese Umweltgrößen nicht innerhalb dieses Wertebereichs, zum Beispiel weil die Umweltgröße (wie beispielsweise die Temperatur) viel zu niedrig ist oder zu hoch ist (wie beispielsweise die Staubdichte), so gibt das Testgerät die Messungen nicht frei und zeigt einem Benutzer an, warum dass das Testgerät die Messungen nicht freigibt.
   Da das Umweltinformationssystem vorzugsweise mehrere Sensoren bzw. Multisensoren aufweist, mit denen verschiedene Umweltgrößen erfasst werden können, ist es auch möglich, dass das Testgerät 1 die Messungen eines Schüttguts erst dann nicht freigibt, wenn mehrere vordefinierte Umweltgrößen (zum Beispiel die Temperatur und die Dichte an Staubpartikeln) nicht in einem vorgegebenen Wertebereich liegen. Alternativ kann auch vorgesehen sein, dass alle Umweltgrößen, die von dem Umweltinformationssystem erfasst werden, in einem vordefinierten Wertebereich liegen müssen, damit das Testgerät 1 die Messungen an dem Schüttgut freigibt.
   Die vorgegeben Wertebereiche für eine jede Umweltgröße sind in einem an dem Testgeräts 1 angeordneten Computersystem (externes Computersystem) oder in einem innerhalb des Testgeräts 1 angeordnetes Computersystem (internes Computersystem) hinterlegt. Bei diesem Computersystem kann es sich um einen PC, der an dem Testgerät 1 angeordnet ist, oder um ein in dem Testgerät 1 integriertes Computersystem, das im unteren Abschnitt 2 angeordnet sein kann, handeln. Ein solches Computersystem ist in den Figuren jedoch nicht gezeigt. Da das Computersystem an oder in dem Testgerät 1 angeordnet ist, ist das Computersystem Teil des Testgeräts 1.
   Das Testgerät sowie das Computersystem, ob intern oder extern, können auch als Anordnung definiert werden. Das Computersystem besteht aus Hardware sowie Software, wobei die Software die Auswertung durchführt.
   Im Folgenden wird jedoch nur davon gesprochen, dass das Computersystem die Auswertung durchführt.
   Um zu prüfen, ob die gemessenen Umweltgrößen innerhalb des vorgegebenen Wertebereichs liegen, werden diese in dem Umweltinformationssystem in weiterverarbeitbare elektrische Signale umgewandelt und diese elektrischen Signale an das Computersystem weitergeleitet. Das Computersystem führt sodann eine Auswertung durch, indem es anhand der aus den Signalen erhaltenen Daten prüft, ob die ermittelten Umweltgrößen innerhalb der vorgegeben Wertebereiche liegen oder nicht.
   Diese hinterlegten Wertebereiche sind somit einem bestimmten Schüttgutsorte zugeordnet.
   Entscheidet sich ein Benutzer das Schüttgut zu vermessen, obwohl das Testgerät 1 die Messungen nicht freigegeben hat, kann dieser Benutzer das Testgerät 1 für die Messungen dennoch freischalten.
2. Während das Schüttgut einer bestimmten Sorte vermessen wird, werden die Umweltgrößen durch die entsprechenden Sensoren des Umweltinformationssystems ständig, vorzugsweise in regelmäßigen Abständen, zumindest aber zu Anfang der Messungen, erfasst. Die so ermittelten Umweltgrößen fließen in die Messungen ein. Dazu werden die gemessenen Umweltgrößen in weiterverarbeitbare elektrische Signale umgewandelt und an das Computersystem weitergeleitet. Wird nun Schüttgut mittels der Messvorrichtung 23 vermessen, so werden für jedes Schüttgut verschiedene Messwerte für bestimmte Eigenschaften bzw. Größen, wie zum Beispiel die Länge des Schüttguts, die Breite des Schüttguts, die Masse, die Härte und/oder die Zusammensetzung, erhalten. Diese Messwerte werden ebenfalls in dem Computersystem hinterlegt. Dazu werden die Messwerte in elektrische Signale umgewandelt und an das Computersystem weitergeleitet. Das Computersystem, bestehend aus Hardware und Software, wandelt die erhaltenen elektrischen Signale in Daten um, die dann in dem Computersystem hinterlegt werden.
   Da das Schüttgut einer bestimmten Sorte in dem Testgerät 1 bei ganz bestimmten Umweltbedingungen (= allen gemessenen Umweltgrößen) vermessen wurde, können die erhaltenen Messwerte für diese Schüttgutsorte (hier: Oblongs) diesen Umweltbedingungen zugeordnet werden, weil in dem Computersystem die Umweltbedingungen in Form von Umweltgrößen hinterlegt sind.
   Anschließend kann in dem Testgerät 1 Schüttgut einer anderen Sorte vermessen werden, wobei bei den Messungen wiederum die Umweltgrößen gemessen werden, womit auch für diese andere Schüttgutsorte die erhaltenen Messwerte den gegebenen Umweltbedingungen zugeordnet werden können.
   Dadurch werden Messungen einer bestimmten Schüttgutsorte mit einem bestimmten Testgerätetyps vergleichbar und reproduzierbar gemacht. So ist nämlich stets bekannt, welche Messergebnisse bei welchen Umweltbedingungen für diesen Testgerätetyp erhalten wurden.
   Damit spielt es beispielsweise keine Rolle mehr, ob ein Schüttgut einer bestimmten Sorte in einem tropischen oder einem arktischen Gebiet durchgeführt wurden, weil die erhaltenen Messwerte diese Schüttgutsorte und einem Testgerät des gleichen Typs miteinander verglichen werden können. Ferner spielt es keine Rolle, an welchen Orten oder auf welchem Untergrund das Testgerät. So kann das Testgerät beispielsweise neben einer großen laufenden Tablettiermaschine stehen, durch die das Testgerät Erschütterungen ausgesetzt ist.
   Da es sein kann, dass an verschiedenen Orten des Testgeräts sich die gemessenen, gleichen Umweltgrößen (zum Beispiel die Temperatur oder die Staubdichte) voneinander stark unterscheiden, kann es sinnvoll sein, mehrere Sensoren oder Multisensoren in dem Testgerät anzuordnen, mit denen die gleichen Umweltgrößen gemessen werden, womit ein Mittelwert für eine bestimmte Umweltgröße gebildet werden kann. Dadurch fließt in eine bestimmte Messgröße, die durch das Vermessen des Schüttguts erhalten wurde, nicht mit nur eine Umweltgröße, die anderen bestimmten Ort gemessen wurde, ein, sondern es fließt ein Mittelwert dieser Umweltgröße ein.

### Bezugszeichenliste

- 1: Testgerät
- 2: Unterer Abschnitt
- 3: Anordnung zur Vereinzelung
- 4: Gehäuse
- 5: Vibrationsplatte
- 6: Vorratsbehälter
- 7: Öffnung
- 8: Normanschluss
- 9: Schüttgutzuführung
- 10: Oberer Bereich
- 11: Transportvorrichtung
- 12: Vibrationsantrieb
- 13: Pfeil
- 14: Pfeil
- 15: Boden
- 16 bis 27: Kammern
- 23: Messvorrichtung
- 28: Öffnung
- 29: Schüttgut
- 30: Schüttgut
- 31: Schüttgut
- 32: Schüttgut
- 33: Schüttgut
- 34: Messstation/Waage
- 35: Abdeckung
- 36: Arbeitsraum
- 37: Außenumgebung

## Patentansprüche

1. Testgerät (1) zum Vermessen von kleinvolumigem Schüttgut (29 bis 33), wobei das Testgerät (1) in einem Arbeitsraum (36; 36 und 37) eine Messvorrichtung (23) aufweist, die mindestens eine Messstation (34) umfasst, in der Schüttgut (29 bis 33) vermessen werden kann, **dadurch gekennzeichnet, dass** das Testgerät (1) in dem Arbeitsraum (36; 36 und 37) ein Umweltinformationssystem aufweist, wobei das Umweltinformationssystem mindestens einen Sensor und/oder mindestens einen Multisensor aufweist, wobei mit jedem Sensor eine bestimmte Umweltgröße gemessen werden kann und/oder mit jedem Multisensor verschiedene Umweltgrößen gemessen werden können und diese mindestens eine gemessene Umweltgröße in ein elektrisches Signal umwandelbar ist, wobei das Testgerät (1) einen unteren Abschnitt (2) aufweist, auf dem eine Anordnung zur Vereinzelung (3) angeordnet ist.

2. Testgerät nach Patentanspruch 1, **dadurch gekennzeichnet, dass** die im Arbeitsraum (36; 36 und 37) angeordnete Messvorrichtung (23) mehrere Messstationen (34) aufweist.

3. Testgerät nach den Patentansprüchen 1, **dadurch gekennzeichnet, dass** das Testgerät (1) ein Computersystem umfasst, wobei das Testgerät (1) und das Computersystem eine Anordnung bilden, wobei das Computersystem anhand der aus dem mindestens einen elektrischen Signal erhaltenen Daten prüft, ob die zumindest eine ermittelte Umweltgröße innerhalb eines vorgegeben Wertebereichs liegt oder nicht und/oder das Computersystem anhand der aus dem mindestens einen elektrischen Signal erhaltenen Daten die erhaltenen Messwerte für einen bestimmten Schüttgutsorte der mindestens einen gemessenen Umweltgröße zuordnet.
